# EUROPÄISCHE PATENTSCHRIFT

(11) **EP 0 584 757 B1**
(45) Veröffentlichungstag und Bekanntmachung des Hinweises auf die Patenterteilung: **19.11.1997**
(21) Anmeldenummer: 93113420.9
(22) Anmeldetag: 23.08.1993
(51) Int. Cl.: A61F 13/15

(54) **Wegwerfwindeln**
Disposable nappies
Couches-culottes à jeter

(30) Priorität: 24.08.1992 JP 224342/92
(43) Veröffentlichungstag der Anmeldung: 02.03.1994
(73) Patentinhaber: UNI-CHARM CORPORATION, Kawanoe-shi Ehime-ken (JP)
(72) Erfinder: Fujioka, Yoshihisa, Mitoyo-gun, Kegawa-ken (JP); Mukai, Hirotomo, Kawanoe-shi, Ehime-ken (JP)
(74) Vertreter: Sperling, Rüdiger, Dipl.-Ing.

(56) Entgegenhaltungen:
- FR-A- 2 517 524
- FR-A- 2 517 525
- US-A- 2 654 367
- US-A- 3 860 003

## Beschreibung

### DER ERFINDUNG ZUGRUNDE LIEGENDER STAND DER TECHNIK

Die vorliegende Erfindung betrifft eine Wegwerfwindel.

Ein typisches Beispiel einer Wegwerfwindel des offenen Typs mit Bandbefestigungseinrichtungen in der Ebene der Hüftlinie, die zum Befestigen eines Vorderteils und eines Hinterteils aneinander verwendet werden, ist im japanischen Patent Nr. 1977-40267 aufgezeigt. Dieses Beispiel umfaßt eine flüssigkeitsdurchlässige Decklage, eine flüssigkeitsundurchlässige Außenlage und eine zwischen diese gelegte, flüssigkeitsabsorbierende Platte, wobei durch die sich über die seitlich gegenüberliegenden Seiten der Platte hinaus erstreckenden Abschnitte der Decklage und der Außenlage zwei Seitenklappen gebildet sind und die jeweiligen Seitenklappen in der Schrittebene mit Ausschnitten versehen sind, die zur Bildung von Beinöffnungen bestimmt sind, um die die jeweiligen Seitenklappen mit elastischen Elementen versehen sind, die zum Abdichten der Seitenklappen um das jeweilige Bein des Trägers dienen, und wobei der Hinterteil an einander seitlich gegenüberliegenden Seiten mit Bandbefestigern versehen ist, die zum Befestigen des Hinterteils am Vorderteil verwendet werden.

Bei der typischen, in vorstehend bezeichnetem japanischen Patent Nr. 1977-40267 aufgezeigten Windel verringern die in den einander gegenüberliegenden Seiten des Schrittbereiches zur verbesserten Anpassung der Windel an den Körper des Trägers ausgebildeten Ausschnitte notwendigerweise die Breite des Schrittbereiches, und es ist praktisch unmöglich, daß der Schrittbereich die Oberschenkel des Trägers vollständig umgibt. Der auf diese Weise breitenreduzierte Schrittbereich vermindert unweigerlich die Fähigkeit des Schrittbereiches, flüssige Ausscheidungen aufzunehmen, insbesondere treten flüssige Ausscheidungen ohne weiteres entlang den einander gegenüberliegenden Seitenrändern des Schrittbereiches aus.

Allgemein verläuft in der bekannten Windel des in vorstehend bezeichnetem japanischen Patent aufgezeigten Typs eine Faltlinie des Schrittbereiches, die einer Grenzlinie des Vorder- und des Hinterteils entspricht, horizontal parallel zur Hüftlinie. Darüberhinaus weist die flüssigkeitsabsorbierende Platte sogenannte halbstarre Eigenschaften auf, da sie oftmals eine mehr oder weniger komprimierte Ansammlung von Faserpulpe und die Ober- und Unterfläche dieser Ansammlung bedeckende Seidenpapierlagen umfaßt. Demgemäß kann sich der Schrittbereich der Windel nicht an den entsprechenden Bereich des Körpers des Trägers anpassen, womit nicht nur dem Träger das Gefühl mangelnder Paßform vermittelt wird, sondern auch das Austreten von Ausscheidungen verursacht wird.

Aus der FR-A-2,517,525 ist eine Wegwerfwindel mit Hilfsplatte bekannt.

### KURZBESCHREIBUNG DER ERFINDUNG

Im Hinblick auf das vorstehend erwähnte Problem ist es eine Hauptaufgabe der Erfindung, eine Wegwerfwindel mit einem in der Weise aufgebauten Schrittbereich aufzuzeigen, daß der Vorteil einer Windel des offenen Typs mit dem Vorteil einer Windel des Kurze-Höschen-Typs verbindet, die das jeweilige Bein eines Trägers vollständig umgebende Bereiche aufweist, und dadurch die Nachteile der vorstehend erwähnten bekannten Windel zu vermeiden.

Der Vorteil der Windel des offenen Typs liegt darin, daß die Windel einstellbar um eine Hüftöffnung wie auch um Beinöffnungen verengt werden kann, und der Vorteil der Windel des Kurze-Höschen-Typs liegt darin, daß die Windel Hosenbeine aufweist, die wenigstens die Innenseiten der Oberschenkel des Trägers bedecken, und daß der Schrittbereich größer als bei einer bekannten Windel dimensioniert sein kann.

Zur Lösung der vorstehend dargelegten Aufgabe besteht die Erfindung allgemein in einer Wegwerfwindel gemäß Anspruch 1.

Vorzugsweise ist der durch die konvex gekrümmte Schweißlinie gebildete Schrittbereich entlang seinem äußeren Rand mit einem entsprechend gekrümmten Ausschnitt versehen.

Bei dem wie vorstehend angegeben gemäß der Erfindung aufgebauten Hygieneartikel wird die flüssigkeitsabsorbierende Hilfsplatte zuverlässig in der Mitte des Schrittbereiches des Trägers ausgerichtet und einander seitlich gegenüberliegende Seiten des Schrittbereiches bedecken jeweils wenigstens die Innenseiten der Oberschenkel des Trägers. Auf diese Weise bedeckt der Schrittbereich der Windel im wesentlichen den gesamten Schritt des Trägers.

### KURZBESCHREIBUNG DER FIGUREN

Die Erfindung wird anhand eines Beispiels unter Bezug auf die beiliegenden Figuren erläutert, wobei
- Fig. 1: eine Vorderansicht einer Ausführungsform der gemäß der Lehre der Erfindung aufgebauten Windel ist; und
- Fig. 2: eine schematische Schnittdarstellung entlang einer Linie X-X in Fig. 1 ist.

### BESCHREIBUNG DER BEVORZUGTEN AUSFÜHRUNGSFORM

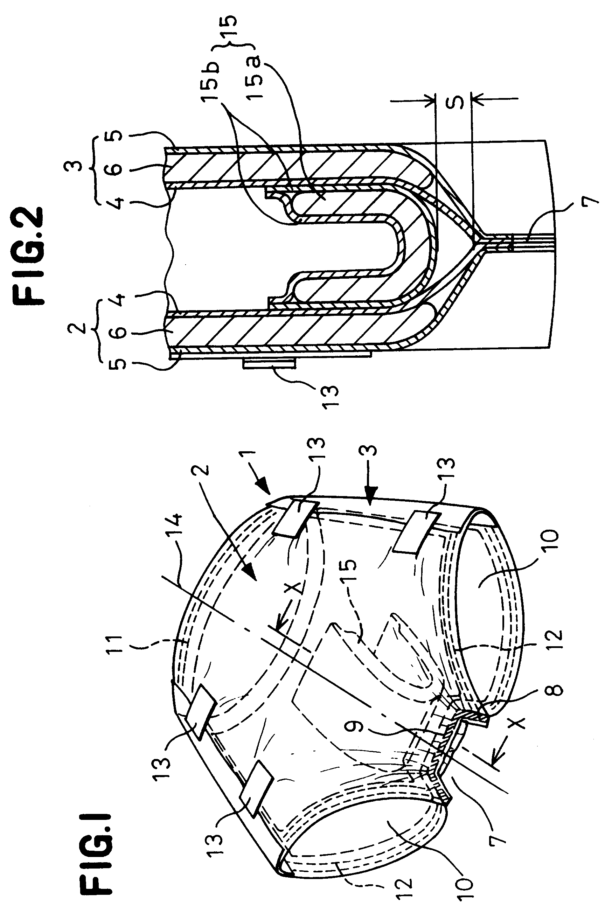

Wie Fig. 1 und 2 zeigen, umfaßt die Grundstruktur 1 einer Windel einen Vorderteil 2 und einen Hinterteil 3. Der Hinterteil 3 ist in Querrichtung größer dimensioniert als der Vorderteil 2. Der Vorderteil 2 und der Hinterteil 3 umfassen eine flüssigkeitsdurchlässige Decklage 4, eine flüssigkeitsundurchlässige Außenlage 5 und eine zwischen die Deck- und die Außenlage 4, 5 gelegte, flüssigkeitsabsorbierende Platte 6.

Die unteren Enden des Vorderteils 2 und des Hinterteils 3 sind an Mittelpositionen mit konvex auf die Hüftlinien des Vorderteils 2 und des Hinterteils 3 zu gekrümmten Ausschnitten 7 versehen. Der Vorderteil 2 und der Hinterteil 3 sind entlang einer parallel zu den Ausschnitten 7 verlaufenden thermischen oder Ultraschall-Schweißlinie 8 miteinander verschweißt, so daß schmale Ränder der Ausschnitte 7 unverschweißt belassen werden. Größe, Form und Krümmungsradius der Schweißlinie 8 kann in Abhängigkeit davon, ob die Windel für Erwachsene oder für Babies vorgesehen ist, in geeigneter Weise ausgewählt werden, solange die Schweißlinie 8 auf die Hüftlinien des Vorder- und des Hinterteils 2, 3 zu gekrümmt ist und wenigstens die Innenseiten von jeweiligen Beinöffnungen 10 sich über einen Scheitelpunkt 9 der konvex gekrümmten Schweißlinie 8 nach unten erstrecken.

In Umfangsrichtung dehnbare elastische Elemente 11, 12 sind zwischen die jeweiligen Ränder der Deck- und der Außenlage 4, 5 gelegt, die sich über die Platte 6 um die Hüftöffnung und die jeweiligen Beinöffnungen hinaus erstrecken, und diese Ränder werden durch Heißschmelzkleber oder die Schweißmittel miteinander verbunden. Einander seitlich gegenüberliegende Seitenränder der Deck- und der Außenlage 4, 5, die sich über die Platte 6 hinaus erstrecken, sind ebenfalls in derselben Weise miteinander verbunden und der Hinterteil 3 ist an einander seitlich gegenüberliegenden Seitenrändern mit mehreren Befestigungseinrichtungen 13 versehen, die jeweils eine Bandbefestigung umfassen, auf die auf einer Seite druckempfindlicher Klebstoff aufgetragen ist, durch den die einander seitlich gegenüberliegenden Seiten des Hinterteils 3 an den entsprechenden Seiten des Vorderteils 2 befestigt werden.

Während die einander seitlich gegenüberliegenden Seitenränder und unteren Ränder um die jeweiligen Beinöffnungen des Vorder- und des Hinterteils 2, 3 so dargestellt sind, daß sie weder parallel noch senkrecht zu einer vertikalen Achse 14 verlaufen, können innerhalb des Umfangs der Erfindung diese Seitenränder parallel zur vertikalen Achse 14 verlaufen und diese unteren Ränder um die jeweiligen Beinöffnungen können senkrecht zur vertikalen Achse 14 verlaufen.

Die Grundstruktur 1 der Windel ist auf der Oberfläche des Schrittbereiches mit einer flüssigkeitsabsorbierenden Hilfsplatte 15 versehen, die in Längsrichtung der Grundstruktur 1 der Windel länglich aufgebaut ist. Die Hilfsplatte 15 umfaßt einen flüssigkeitsabsorbierenden Kern 15a, der mit flüssigkeitsdurchlässigen Lagen 15b bedeckt ist, und ist wenigstens an ihren in Längsrichtung entgegengesetzten Enden mit der Oberfläche der Decklage 4 mittels eines Klebstoffes verbunden. Ist die Grundstruktur 1 der Windel einem Träger angelegt, so ist die Hilfsplatte 15 zusammen mit der Grundstruktur 1 der Windel in U-Form gekrümmt. Die Hilfsplatte 15 ist vorzugsweise mit der Grundstruktur 1 der Windel in einer Weise verbunden, daß die dergestalt gekrümmte Hilfsplatte 15 schwimmend oberhalb der Oberfläche des Schrittbereiches der Grundstruktur liegt, und genauer ausgedrückt ist die äußere Unterseite der gekrümmten Hilfsplatte 15 mit einem Abstand S von wenigstens 10 mm von der Oberfläche des Schrittbereiches der Grundstruktur beabstandet. Es ist jedoch innerhalb des Umfangs der Erfindung auch möglich, die Hilfsplatte 15 so anzuordnen, daß die Unterseite der Hilfsplatte 15 auch in gekrümmtem Zustand in Berührung mit der Oberfläche des Schrittbereiches der Windel verbleibt.

Die untere Lage der Hilfsplatte 15 kann eine flüssigkeitsundurchlässige Lage umfassen, und/oder die Hilfsplatte 15 kann dehnbare, von seitlich einander gegenüberliegenden Seiten derselben ausgehende Seitenklappen aufweisen, ohne vom Umfang der Erfindung abzuweichen.

Die Grundstruktur 1 der Windel ist an den einander seitlich gegenüberliegenden Seiten und daher an der Hüftöffnung wie auch den Beinöffnungen offen und mit Hilfe der in Fig. 1 dargestellten Befestigungseinrichtungen werden die seitlich gegenüberliegenden Seiten geschlossen und folglich werden die Hüftöffnung wie auch die Beinöffnungen ebenfalls geschlossen. Die Größen dieser Öffnungen sind von einer Überlappungsbreite des Vorderteils 2 und des Hinterteils 3 abhängig und die Überlappungsbreite ist wiederum von der Größe der Hüfte und Beine (Oberschenkel) des einzelnen Trägers abhängig.

Die Bestandteile der Windel 1 können aus allgemein in der bekannten Windel verwendeten Materialien hergestellt sein. Beispielsweise kann die Decklage 4 aus Vliesstoff, die Außenlage 5 aus Kunststoffolie, die Platte 6 aus Faserpulpe, gemischt mit einem hochabsorbierenden Polymer, hergestellt sein, die elastischen Elemente 11, 12 können aus natürlichem oder synthetischem Gummi und der Trägerstoff der Befestigungseinrichtung 13 kann aus einem qualitativ hochwertigen Papier oder einem Schichtstoff aus Vliesstoff und Kunststoffolie hergestellt sein. Der Kern 15a der Platte 15 kann aus Faserpulpe, gemischt mit einem hochabsorbierenden Polymer, und die Lage 15b, die den Kern 15a bedeckt, kann aus Vliesstoff hergestellt sein.

Bei der gemäß der Erfindung wie vorstehend beschrieben aufgebauten Windel führt die Bildung der Ausschnitte an gegenüberliegenden Seiten des Schrittbereiches zur Bildung der Beinöffnungen niemals zu einer inakzeptabel schmalen Breite des Schrittbereiches, da die konvex in Richtung der Hüftlinie gekrümmte Schweißlinie die Dimensionierung der Breite des Schrittbereiches, die zwischen den gegenüberliegenden Seitenrändern des Schrittbereiches bestimmt ist, in angemessener Größe ermöglicht. Zusätzlich erlaubt die Bildung einer derartigen Schweißlinie, daß der Schrittbereich wenigstens die Innenseiten der jeweiligen Oberschenkel bedeckt, und das Anordnen der flüssigkeitsabsorbierenden Hilfsplatte über dem Schrittbereich erlaubt es, daß der Schrittbereich flüssige Ausscheidungen in ausreichendem Maße absorbiert, um das Austreten von flüssigen Ausscheidungen entlang den seitlich gegenüberliegenden Seitenrändern des Schrittbereiches zu verhindern, da das absorbierende Material auf der Schweißlinie vorhanden ist, entlang der der getrennt geformte Vorder- und Hinterteil miteinander verschweißt sind.

## Patentansprüche

1. Wegwerfwindel mit einem Vorder- (2) und einem Hinterteil (3), die jeweils eine flüssigkeitsdurchlässige Decklage (4), eine flüssigkeitsundurchlässige Außenlage (5) und eine zwischen diese gelegte flüssigkeitsabsorbierende Platte (6) umfassen und nahe an unteren Enden an einer konvex in Richtung auf eine Hüftlinie des Vorder- (2) und des Hinterteils (3) zu gekrümmten Schweißlinie (8) miteinander verschweißt sind, um so einen Schrittbereich zu bilden, in dem eine flüssigkeitsabsorbierende Hilfsplatte (15), die in Längsrichtung des Vorder- (2) und des Hinterteils (3) länglich ausgebildet ist, vorgesehen ist, **dadurch gekennzeichnet**, daß die flüssigkeitsabsorbierende Hilfsplatte (15) wenigstens an ihren in Längsrichtung entgegengesetzten Enden derart mit der Oberfläche des Schrittbereichs verbunden ist, daß ihre äußere Unterseite in einem Abstand (S) oberhalb der Oberfläche des Schrittbereiches liegt,
und/oder
wenigstens an ihren in Längsrichtung entgegengesetzten Enden mit der Oberfläche des Schrittbereichs verbunden ist und eine flüssigkeitsundurchlässige untere Lage enthält.

2. Wegwerfwindel nach Anspruch 1, **dadurch gekennzeichnet**, daß der Abstand (S), um den die äußere Unterseite der flüssigkeitsabsorbierende Hilfsplatte (15) ggf. oberhalb der Oberfläche des Schrittbereiches liegt, wenigstens 10 mm beträgt.

3. Wegwerfwindel nach einem der vorhergehenden Ansprüche, **dadurch gekennzeichnet**, daß der durch die konvex gekrümmte Schweißlinie (8) gebildete Schrittbereich entlang seinem äußeren Rand mit einem entsprechend gekrümmten Ausschnitt (7) versehen ist.

4. Wegwerfwindel nach einem der vorhergehenden Ansprüche, **dadurch gekennzeichnet**, daß der Hinterteil (3) an einander seitlich gegenüber liegenden Seitenrändern mit jeweils mehreren Befestigungseinrichtungen (13) versehen ist, durch den die einander seitlich gegenüberliegenden Seiten des Hinterteils (3) an den entsprechenden Seiten des Vorderteils (2) befestigbar sind.

5. Wegwerfwindel nach Anspruch 4, **dadurch gekennzeichnet**, daß die Befestigungseinrichtungen (13) Bandbefestigungen enthalten, auf die auf einer Seite drcukempfindlicher Klebstoff aufgetragen ist, durch den die einander seitlich gegenüberliegenden Seiten des Hinterteils (3) an den entsprechenden Seiten des Vorderteils (2) befestigbar sind.

6. Wegwerfwindel nach einem der vorhergehenden Ansprüche, **dadurch gekennzeichnet**, daß sich wenigstens die Innenseiten von jeweiligen Beinöfffnungen (10) über einen Scheitelpunkt (9) der konvex gekrümmten Schweißlinie (8) nach unten erstrecken.

7. Wegwerfwindel nach einem der vorhergehenden Ansprüche, **dadurch gekennzeichnet**, daß der Vorderteil (2) und der Hinterteil (3) getrennt voneinander geformt sind.

8. Wegwerfwindel nach einem der vorhergehenden Ansprüche, **dadurch gekennzeichnet**, daß die flüssigkeitsabsorbierende Hilfsplatte (15) in ihrer Längsausdehnung in einer U-Form gekrümmt ist.

9. Wegwerfwindel nach einem der vorhergehenden Ansprüche, **dadurch gekennzeichnet**, daß die Hilfsplatte (15) dehnbare, von seitlich einander gegenüberliegenden Seiten derselben ausgehende Seitenklappen aufweist.

## Claims

1. Disposable nappy with a forward (2) and a rear portion (3), respectively comprising a fluid-permeable cover layer (4), a fluid-impermeable outer layer (5) and a fluid-absorbing panel (6) placed between these, and are welded together close to lower ends at a weld line (8) curved in a convex manner in the direction of a hip line of the forward (2) and of the rear portion (3), in order in this way to form a crotch area in which there is provided a fluid-absorbing secondary panel (15) which is formed in elongate fashion in the longitudinal direction of the forward (2) and of the rear portion (3), characterised in that
the fluid-absorbing secondary panel (15) is connected at least at its ends opposed in the longitudinal direction to the surface of the crotch area in such a way that its outer underside lies at a spacing (S) above the surface of the crotch area,
and/or
is connected at least at its ends opposed in the longitudinal direction to the surface of the crotch area, and includes a fluid-impermeable lower layer.

2. Disposable nappy according to claim 1, characterised in that the spacing (S) at which the outer lower side of the fluid-absorbing secondary panel (15) if necessary lies above the surface of the crotch area, comes to at least 10 mm.

3. Disposable nappy according to one of the preceding claims, characterised in that the crotch area formed by the convexly curved weld line (8) is provided along its outer edge with a correspondingly curved cut-out portion (7).

4. Disposable nappy according to one of the preceding claims, characterised in that the rear portion (3) is provided, at lateral edges lying mutually opposite one another, respectively with a plurality of fastening devices (13), by means of which the edges lying mutually opposite one another of the rear portion (3) are attachable to the corresponding sides of the forward portion (2).

5. Disposable nappy according to claim 4, characterised in that the fastening devices (13) include strip fastening devices upon which there is applied on one side pressure-sensitive adhesive, by means of which the sides of the rear portion (3) lying mutually opposite one another are attachable to the corresponding sides of the forward portion (2).

6. Disposable nappy according to one of the preceding claims, characterised in that at least the inner sides of respective leg apertures (10) extend downwards via an apex point (9) of the convexly curved weld line (8).

7. Disposable nappy according to one of the preceding claims, characterised in that the forward portion (2) and the rear portion (3) are moulded separately from one another.

8. Disposable nappy according to one of the preceding claims, characterised in that the fluid-absorbing secondary panel (15) is curved in a 'U' shape in the extension of its length.

9. Disposable nappy according to one of the preceding claims, characterised in that the secondary panel (15) has expandable side flaps proceeding from sides of the same lying mutually opposite one another laterally.

## Revendications

1. Lange jetable, comprenant une partie avant (2) et une partie arrière (3), lesquelles comportent, respectivement, une mince couche de revêtement perméable aux liquides (4), une mince couche extérieure imperméable aux liquides (5) et une couche (6) plus épaisse absorbant les liquides, interposée entre ces dernières, lesdites couches minces étant soudées l'une à l'autre à proximité d'extrémités inférieures, sur une ligne de soudure (8) s'incurvant avec une courbure convexe dans la direction d'une ligne de hanches des parties avant (2) et arrière (3), pour former ainsi une zone d'entrejambe dans laquelle est prévue une garniture auxiliaire (15) absorbant les liquides qui présente une forme allongée dans le sens de la longueur des parties avant (2) et arrière (3), caractérisé en ce que la garniture auxiliaire (15) absorbant les liquides est raccordée, au moins à ses extrémités longitudinales opposées, avec la surface de la zone d'entrejambe, de telle façon que sa face inférieure extérieure se situe à une certaine distance (S) au-dessus de la surface de la zone d'entrejambe, et/ou en ce qu'elle est raccordée, au moins à ses extrémités longitudinales opposées, avec la surface de la zone d'entrejambe, et comprend une couche inférieure imperméable aux liquides.

2. Lange jetable selon la revendication 1, caractérisé en ce que la distance (S) dont la face inférieure extérieure de la garniture auxiliaire (15) absorbant les liquides est éventuellement écartée du dessus de la surface de la zone d'entrejambe, est d'au moins 10 mm.

3. Lange jetable selon l'une des revendications précédentes, caractérisé en ce que la zone d'entrejambe formée par la ligne de soudure (8) d'incurvation convexe est pourvue, sur son bord extérieur, d'une découpure (7) d'incurvation correspondante.

4. Lange jetable selon l'une des revendications précédentes, caractérisé en ce que la partie arrière (3) est dotée, sur des bords latéraux mutuellement opposés, de plusieurs éléments de fixation (13) permettant d'assembler les faces latérales mutuellement opposées de la partie arrière (3) aux faces correspondantes de la partie avant (2).

5. Lange jetable selon la revendication 4, caractérisé en ce que les éléments de fixation (13) comprennent des bandes de fixation sur lesquelles a été appliqué, sur une face, un adhésif sensible à la pression, permettant d'assembler les faces latérales mutuellement opposées de la partie arrière (3) aux faces correspondantes de la partie avant (2).

6. Lange jetable selon l'une des revendications précédentes, caractérisé en ce qu'au moins les faces intérieures d'ouvertures de passages des jambes (10) s'étendent vers le bas en passant par un point culminant (9) de la ligne de soudure (8) d'incurvation convexe.

7. Lange jetable selon l'une des revendications précédentes, caractérisé en ce que la partie avant (2) et la partie arrière (3) sont formées séparément l'une de l'autre.

8. Lange jetable selon l'une des revendications précédentes, caractérisé en ce que la garniture auxiliaire (15) absorbant les liquides est incurvée en forme de U dans son étendue longitudinale.

9. Lange jetable selon l'une des revendications précédentes, caractérisé en ce que la garniture auxiliaire (15) contient des rabats latéraux extensibles, qui partent de faces latérales mutuellement opposées de cette dernière.
